# EUROPEAN PATENT SPECIFICATION

(11) **EP 0 562 132 B1**
(45) Date of publication and mention of the grant of the patent: **03.06.1998**
(21) Application number: 92105000.1
(22) Date of filing: 23.03.1992
(51) Int. Cl.: C12P 21/08, C12N 5/24, A61K 39/395

(54) **Monoclonal anti-tetanus toxin antibodies and pharmaceutical compositions containing them**
Monoklonale Antikörper gegen Tetanustoxin und sie enthaltende pharmazeutische Zusammensetzungen
Anticorps monoclonaux contre la toxine tétanique et compositions pharmaceutiques les contenant

(43) Date of publication of application: 29.09.1993
(73) Proprietor: SCHWEIZERISCHES SERUM- & IMPFINSTITUT BERN, CH-3001 Bern (CH)
(72) Inventor: Lang, Alois B., Dr., CH-3627 Heimberg (CH)
(74) Representative: VOSSIUS & PARTNER

(56) References cited:
- EP-A- 0 157 574
- EUROPEAN JOURNAL OF EPIDEMIOLOGY vol. 6, no. 4, December 1990, ROME, ITALY pages 386 - 397 M. KAMEI ET AL. 'Establishment of stable mouse/human-human hybrid cell lines producing large amounts of anti-tetanus human monoclonal antibodies with high neutralizing activity.'
- EUROPEAN JOURNAL OF EPIDEMIOLOGY vol. 8, no. 1, January 1992, ROME, ITALY pages 1 - 8 M. MATSUDA ET AL. 'Characteristics of toxin-neutalization by anti-tetanus human monoclonal antibodies directed against the three functional domains (A), (B) and (C) of the tetanus toxin molecule and a reliable method for evaluating the protective effects of monoclonal antibodies.'
- ABSTRACTS OF PAPERS - AMERICAN CHEMICAL SOCIETY. vol. 200, no. 1-2, 1990, WASHINGTON DC, US page AGFD M. MATSUDA ET AL. 'Isolation of fragments corresponding to domains (A), (B), (C) of the tetanus toxin molecule and preparation of human anti-tetanus monoclonal antibodies with high neutralizing activity for study of structure-function of tetanus toxin.'
- DATABASE WPIL Week 9014, Derwent Publications Ltd., London, GB; AN 90-104925
- INFECTION AND IMMUNITY vol. 42, no. 3, December 1983, WASHINGTON DC, US pages 942 - 948 J. KENIMER ET AL. 'Monoclonal antibodies as probes of tetanus toxin structure and function.'
- Botulinum and Tetanus Neurotoxins, ed by B.R.DasGupta, Plenum Press, New York, pages 273-286

## Description

The present invention relates to monoclonal anti-tetanus toxin antibodies binding to the light chain of the tetanus toxin and neutralizing the biological activity of the tetanus toxin. Furthermore, the invention relates to cell lines, preferably hybridomas, producing said monoclonal anti-tetanus toxin antibodies. The present invention also relates to a method for the production of said monoclonal anti-tetanus antibodies and to pharmaceutical compositions containing them.

Tetanus is a neurologic disease with severe muscular spasms caused by the neurotoxin produced by Clostridium tetani in a contaminated wound. Tetanus accounts world-wide for approximately 50% of neonatal deaths and 25% of infant deaths.
Tetanus toxin is synthesized by the bacterium as an inactive single chain polypeptide that is converted to an active dichain molecule, consisting of a light chain of approximately 50'000 molecular weight and a heavy chain of approximately 100'000 molecular weight. Three moieties each with a molecular weight of approximately 50'000 of the toxin have been described: the light chain or A-fragment, which is assumed to mediate the toxic activity; the amino terminal moiety or B-fragment of the heavy chain, which has been claimed to be responsible for the internalization of the toxin by its target cells, and the carboxyterminal moiety or C-fragment of the heavy chain which mediates the binding of the toxin to gangliosides present on neuronal cells.
Currently, prophylaxis against tetanus comprises active immunization with a toxoid vaccine and - if indicated as wound management - passive immunization with polyclonal anti-tetanus antibody preparations (TIG). Due to the occurrence of allergic reactions, including serum sickness and shock, heterologous animal antisera have been replaced by homologous human polyclonal antibody preparations. However, these antibodies are isolated from plasma of hyperimmunized donors and, as a consequence, are always afflicted with the risk of potentially fatal - may be not yet known - contamination. Furthermore, the supply and the heterogeneity of polyclonal preparations can be limiting. Human monoclonal antibodies (HmAb) provide a uniform, unlimited source of defined reagents which may overcome most of the limitations of polyclonal preparations. However, so far the generation of appropriate HmAb has not been successful. EP 0 157 574 describes the production of human-human hybridoma having antitetanic antibody producing ability. The large number of different epitopes recognized by polyclonal antibody preparations normally ensures the presence of protective antibodies. However, the isolation of one or a limited number of HmAb providing optimum protection requires the ability to generate and screen a large number of stable, antibody secreting human hybridomas. Because of the ease of obtaining competent B cells from vaccinated persons, tetanus toxin (TT) has been a popular antigen.
Kenimer et al., Infection and Immunity 42 (1983), 942, and JP 2-057195 describe antibodies specific for the A-, B- and C-fragments of the tetanus toxin and showing neutralizing capacity of the tetanus toxin. Despite of the several reports of HmAb specific for TT, relatively little is known about their protective capacities; Kamei et al., Eur. J. Epidemiol. 6 (1990), 386, Simpson et al., J. Pharmacol. Exp. Ther. 254 (1990), 98, Gigliotti and Insel, J. Clin. Invest. 70 (1982), 1306. When analyzed, the capacity of these HmAb was relatively little and inferior to that of commercial polyclonal products; Trabaud et al., APMIS 97 (1989), 671, Ziegler-Heitbrock et al., Hybridoma 5 (1986), 21. Matsuda et al., Eur. J. Epidemiol. 8 (1992), 1 and Matsuda et al., Abs. Pap. Amer. Chem. Soc. 200(1-2) 1990, page AGFD, Abs. no. 53, describe antibodies with binding capacities to the A-, B- and C-fragments of the tetanus toxin showing complete protection of mice from tetanus intoxication. Furthermore, nothing is known about the feasibility of commercially producing such HmAb.

Thus, the technical problem underlying the present invention is to provide monoclonal anti-tetanus toxin antibodies which are capable of replacing polyclonal antibodies in the treatment of tetanus.

The solution to the above technical problem is provided by the embodiments characterized in the claims.

Accordingly, the present invention provides a monoclonal anti-tetanus toxin antibody binding to the light chain of the tetanus toxin and neutralizing the biological activity of the tetanus toxin.
In this context, the term "neutralizing the biological activity of the tetanus toxin" means that the monoclonal anti-tetanus toxin antibodies of the present invention are capable of effectively prophylactically protecting, for instance, human patients from tetanus.

In a preferred embodiment of the present invention, the monoclonal anti-tetanus antibody is a human antibody.

In another preferred embodiment, the monoclonal anti-tetanus toxin antibody of the present invention does not only bind to the light chain of the tetanus toxin (A-fragment) but also to the C-fragment of the heavy chain of the tetanus toxin. These antibodies display a surprisingly high capability of neutralizing the biological activity of the tetanus toxin. Thus, they are particularly well suited for being used in pharmaceutical compositions for the prophylaxis and treatment of tetanus.

In the further preferred embodiment, the monoclonal anti-tetanus toxin antibodies of the present invention have an affinity constant (aK) to said tetanus toxin of at least 1x10⁹ M⁻¹. According to the present invention, this affinity constant was determined by competitive binding assays. The apparent affinity constants (aK) were obtained by determining the reciprocal value of molar concentration resulting in 50% inhibition of the antibody binding.

In a particularly preferred embodiment, the monoclonal anti-tetanus toxin antibodies of the present invention display an affinity constant (aK) to said tetanus toxin of at least 5x10⁹ M⁻¹.

In another embodiment, the monoclonal anti-tetanus toxin antibodies of the present invention have a neutralizing capacity of at least 2IU/100 µg Ig, preferably of at least 4 IU/100µg Ig. According to the present invention, this neutralizing capacity was determined and quantitated by the lethal method in mice, using standard test toxin and standard tetanus antitoxin.

In another particularly preferred embodiment of the present invention, the monoclonal anti-tetanus toxin antibodies display a neutralizing capacity of at least 10 UI/100µg Ig.

In a further preferred embodiment of the present invention, the monoclonal anti-tetanus toxin antibodies belong to the class IgG and preferably to the class IgG1.

Furthermore, the monoclonal anti-tetanus toxin antibodies of the present invention can be chimeric, humanized or bi-specific rearranged monoclonal antibodies.
In this context, the term "chimeric" refers to monoclonal antibodies which are encoded by murine and human gene elements.

The term "humanizing" refers to monoclonal antibodies which consist of binding sites (variable regions or hypervariable regions) encoded by murine gene elements and constant regions or constant regions and framework of the variable regions encoded by human gene elements.

Furthermore, the term "bi-specific rearranged monoclonal antibodies" refers to monoclonal antibodies which consist of two different binding sites each with a different specificity.

Another object of the present invention is to provide cell lines producing the monoclonal anti-tetanus toxin antibodies of the present invention. Examples of such cell lines are hybridomas, EBV-transformed lymphoblastoid cell lines or mammalian cell lines expressing immunoglobulin genes.

In a preferred embodiment, said cell line is a hybridoma. The present invention particularly prefers hybridomas which are obtained by fusing a B cell derivative being capable of producing the monoclonal antibody of the present invention with a human heteromyeloma cell line, such as LA55. LA55 is a heteromyeloma cell line derived from a fusion of human lymphocytes with a mouse myeloma cell line essentially produced as described by Lang and Bruderer; Hybridoma 11 (1992), 99. Of course, the person skilled in the art may also use other human heteromyeloma cell lines in order to produce the monoclonal antibodies of the present invention.

Particularly preferred hybridomas of the present invention are ST11, ST12, ST14, ST15, ST17, ST18 and ST19.

A further object of the present invention is a method for the production of said monoclonal anti-tetanus toxin antibodies which comprises the cultivation of a cell line of the present invention and the isolation of the antibody from the culture.

Another object of the present invention is to provide a pharmaceutical composition containing at least one, preferably at least two monoclonal antibodies of the present invention, optionally in admixture with a pharmaceutically acceptable carrier and/or diluent.

The most preferred embodiment of the present invention is a pharmaceutical composition which contains two monoclonal antibodies of the present invention. As can be taken from the example described below, such pharmaceutical compositions display a remarkably improved neutralizing capacity. Therefore, they are particularly useful for preventing and/or treating tetanus.

In a preferred embodiment of the present invention, the pharmaceutical composition contains a monoclonal anti-tetanus toxin antibody which binds to the light chain of the tetanus toxin, and in addition it contains a monoclonal anti-tetanus toxin antibody which binds to the light chain of the tetanus toxin and also to the C-fragment of the heavy chain of the tetanus toxin.

In another preferred embodiment, the pharmaceutical composition of the present invention is for the passive immunization against tetanus. In this context, the term "passive immunization" refers to the prophylaxis or treatment of tetanus.

The pharmaceutical compositions of the present invention are for the prophylaxis and/or treatment of all kinds of mammalians which may suffer from tetanus. In particular, they are for the prophylaxis and/or treatment of tetanus in human patients.

In a particularly preferred embodiment, the pharmaceutical compositions of the present invention contain each of the monoclonal antibodies in an amount of at least 0.5 mg Ig corresponding to an immunoprotective dosage of at least 250 IU. In this context, the term "immunoprotective dosage" refers to a dosage which provides full protection of the individual being treated against tetanus.

Legends to the figures:

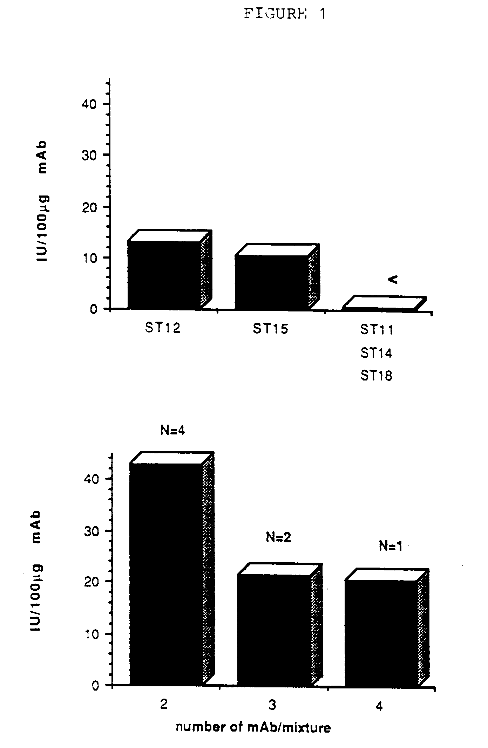

- Figure 1:: Synergistic effect of mixtures of equal amounts of HmAb. Protectivity in mice of ST12 alone or in combination with one or two additional HmAb (ST15, ST11, ST14, ST18) are shown in terms of IU/100µg IgG; shown are the combinations which provided the best protection (ST12/ST15 for combinations of two, ST12/ST15/ST18 for combinations of three).

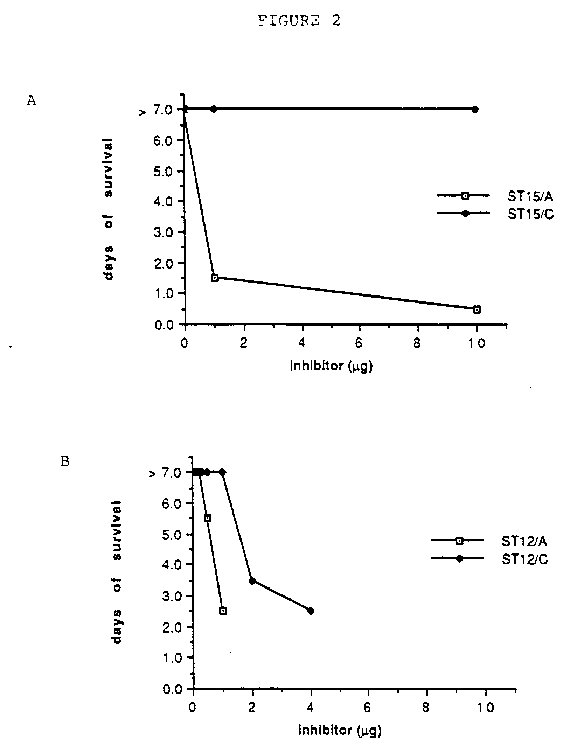

- Figure 2:: Correlation between antibody fine specificity and protective capacity in vivo. Constant amounts of HmAb ST15 (2A), ST12 (2B), were incubated with variable amounts of purified A-fragment or C-fragment and the protective capacity in vivo of these mixtures was tested.

The Example illustrates the invention.

### Example

### The production and characterization of monoclonal anti-tetanus toxin antibodies

(A) Isolation characterization of tetanus toxin (TT) and TT fragments
TT was isolated and purified according to Ozutsumi et al., Appl. Environ. Microbiol. 49 (1985), 939.
Tetanus toxoid was then prepared by formaldehyde detoxification of purified TT. The light chain (A-fragment) was prepared by trypsin treatment of purified TT according to Matsuda and Yoneda, Biochem. Biophys. Res. Comun. 57 (1974), 1257, followed by reduction with dithiothreitol and urea treatment according to Matsuda and Yoneda, Infect. Immun. 12 (1975) 1147. The C-fragment was prepared by papain digestion of purified TT as described by Matsuda and Yoneda in Biochem. Biophys. Res. Commun. 77 (1977), 268. The fragments were purified by gel filtration on an ultragel® AcA44 column (LKB); see Matsuda and Yoneda, Biochem. Biophys. Res. Commun. 57 (1974) 1257. The purity of the fragments was assessed by HPLC on a Zorbax GF-250 column (Dupont) toxicity tests in guinea pigs, infra, and by binding assays, infra.
Applying size exclusion HPLC, no contamination could be detected in the purified TT, A-fragment, and C-fragment preparations. In contrast to whole TT molecules, fragments are not toxic; see Helting and Zwisler, J. Biol. Chem. 252 (1977), 187. Injection of 0.5 ng purified TT was sufficient to kill the guinea pigs. However, 500 ng purified A-fragment or C-fragment did not kill the animals. This indicates that the fragment preparations contain < 0.1% TT.
(B) Production of human monoclonal antibodies
PBL were obtained from healthy volunteers ten days after booster immunization with the tetanus toxoid (DiTe Anatoxal Berne, Switzerland). Human hybridomas were generated essentially as described in Lang et al., Hum. Antibod. Hybridomas 1 (1990), 96 and Lang et al., J. Immunol. 146 (1991), 3160. Briefly, mononuclear cells were separated on Ficoll-Hypaque® and monocytes depleted by adherence to plastic. Nonadherent cells were selected for the presence of anti-TT antibody on their surfaces by Ag-specific panning. Six well plates were coated with 150 µg TT/well diluted in 0.05 M NaHCO₃ buffer, pH 9.6. Plates were incubated over night at 4°C. After blocking the remaining binding sites and washing, PBL were added and incubated for 70 min. at 4°C. The panned cells were EBV-transformed by exposure to EBV containing supernatant. Afterwards cells were washed and distributed into 96 well microtiter plates. Culture supernatants were tested by ELISA (see below) for anti-TT antibodies. Cells from positive wells were subcultured and retested for the secretion of specific anti-TT antibodies by ELISA using toxin, toxoid and TT fragments. Positive cultures were fused to the human heteromyeloma cell line LA55 using the plate fusion technique. After fusion cells were cultured for 24 hrs. in serum free medium (CG-medium, Camon, Labor Service GmbH, Wiesbaden, Germany) supplemented with 1% human serum (Flow Laboratories, Herts, England), followed by exposure to CG-medium containing 50 µM hypoxanthine, 2.5 µg/ml azaserine, and 2.5 µM ouabain (all Sigma Chemical Co., St. Louis, MO). Subsequently, cultures were propagated in serum-free CG-medium. Wells containing hybridomas secreting specific anti-TT antibodies were cloned by limiting dilution.
(C) Detection and purification of anti-TT antibodies
Anti-TT antibodies were detected by standard ELISA methods in microtiter plates (Immulon; Dynatech, Buchs, Switzerland) coated with 10 ug/ml of purified TT, toxoid, or TT fragments diluted in 0.1 M NaHCO₃, pH 9.6. HmAb were affinity purified from culture supernatant on protein A-Sepharose® (Pharmacia, Sweden). IgG was eluted from the column (1.5 ml vol.) with o.1 M citric acid, pH 3.0, The pH of the eluate was adjusted to pH 7.2 with 1 M Tris, pH 9.0. Aliquots were stored at -70°C until use.
(D) Isotype determination and antibody quantitation.
The isotopes of HmAb were determined by ELISA (supra) using heavy and light chain-specific antisera (Tago, Burlingame, CA). The IgG subclasses were detected by ELISA using the murine mAb specific for human IgG1 (clone HP6012), IgG4 (HP6011) (Unipath, Ltd., Bedford, UK) and IgG2 (HP6014 (Sigma Chem.). Antibody concentrations were determined by a radial immunodiffusion assay (LC-Partigen-IgG kit, Behring, Marburg, Germany) or by a sandwich ELISA) (13, 18) using purified HmAb or standard human serum (Behring) to generate standard curves.
(E) Determination of antibody fine specificity and affinity.
Fine specificity of HmAb was determined by ELISA with A-fragment or C-fragment in the solid phase (see above) and by determining antibody affinities for the corresponding fragments. Affinities were determined by competitive binding assays as decribed in Bruderer et al., J. Immunol. Methods 133 (1990) 263.
(F) Characterization of TT specific HmAb.
Of 100 TT-specific HmAb tested, only 7 showed high TT neutralizing activity in mice by primary screening of culture supernatant of the corresponding hybridomas. The reactivity patterns of these neutralizing HmAB and of additional 24 randomly selected and processed HmAb are shown in Table I.

**Table I**

| REACTIVITY PATTERN OF HUMAN MONOCLONAL ANTI-TT ANTIBODIES | | | |
|---|---|---|---|
| Specificity^{a} TT-fragment | Neutralizing capacity^{b} | Number of clones | Distribution^{c} (%) |
| A | - | 23 | 74 |
| A | + | 5 | 16 |
| C | - | 1 | 3 |
| C | + | 0 | 0 |
| AC | - | 0 | 0 |
| AC | + | 2 | 7 |

| | | | |
|---|---|---|---|
| ^{a} (A), affinity for fragment A ≥ 10⁷ M⁻¹, for C < 10⁷ M⁻¹; (C), affinity for fragment A < 10⁷ M⁻¹, for C ≥ 10⁷ M⁻¹; and (AC), affinity for fragment A ≥ 10⁷ M⁻¹, for C ≥ 10⁷ M⁻¹. | | | |
| ^{b} Determined in mice by the initial screening for toxin-neutralizing activity of the HmAb; (+), complete protection, (-), no complete protection. | | | |
| c (%) of clones with the corresponding properties. | | | |

Table 1 demonstrates that most of the clones (90%) exhibit specificity for the A-fragment, but less than 20% of these A-fragment specific HmAb neutralized TT. The C-fragment specific HmAb was not neutralizing, whereas both HmAb which specifically bind both the A- and C-fragment neutralized TT.
An extended characterization of neutralizing HmAb is shown in Table II.

**Table II**

| BINDING PROPERTIES AND TOXIN-NEUTRALIZING ACTIVITY OF HUMAN MONOCLONAL ANTI-TT ANTIBODIES | | | | | | |
|---|---|---|---|---|---|---|
| Clone | Isotype Ig | aK (x10⁹ M⁻¹)^{a} | | | IU/100µg IgG^{b} | |
| | | Toxin | Fr. A | Fr. C | 45 MLD | 450 MLD |
| ST12 | G1/k | 1.3 | 0.83 | 1.9 | >5.5 | 13.2 |
| ST18 | G1/l | 0.33 | 0.052 | <0.01 | nd | <0.5 |
| ST19 | G1/k | 5.2 | 4.1 | 9.9 | nd | nd |
| ST17 | G1/k | 3.2 | 3.9 | <0.01 | 4.4 | <0.5 |
| ST15 | G1/k | 5.6 | 1.9 | <0.01 | >5.5 | 10.5 |
| ST11 | G1/k | 6.2 | 0.57 | <0.01 | >5.5 | <0.5 |
| ST14 | G1/l | 2.6 | 4.1 | <0.01 | 2.3 | <0.5 |

| | | | | | | |
|---|---|---|---|---|---|---|
| ^{a} Affinity constants (aK) for TT and the fragments A and C. | | | | | | |
| ^{b} International Units (IU), values determined using a reference preparation of tetanus anti-toxin, calibrated in IU and a calibrated preparation of test toxin dose of either 45 MLD or 450 MLD; affinity purified HmAb were used. | | | | | | |

It can be taken from Table II that all identified HmAb are of the IgG1 class associated with either Kappa or Lambda chains. The A-fragment specific HmAb exhibited comparable affinities for TT and the A-fragment, whereas they showed no detectable affinity for purified C-fragment. ST12 and ST19 exhibit similar affinities for TT, A- and C-fragment. The affinities range from 3.3 x 10⁸ M⁻¹ to 6.2 x 10⁹ M⁻¹.
(G) The neutralizing activities of the obtained HmAbs in mice
The ability of HmAb to neutralize TT was evaluated by the lethal method in mice (European Pharmacopeia, Tetanus antitoxin for human use, 2nd ed. Saint Ruffine, France: Maisonneuve S.A. (1981), Monograph No. 91) using standard test TT and standard tetanus antitoxin (WHO, International Standards and Reference Reagents, 1990). The minimal lethal dose (MLD) of the standard test toxin was determined from the dose-response curve; see European Pharmacopeia, Supra. The following three assays were used:
(a) Initial screening for toxin-neutralizing activity of HmAb in the culture supernatants of the hybridomas: 3 volumes of culture supernatant containing HmAb was mixed with 1 volume of standard TT solution (90 MLD). Mice surviving 6 days after challenge were considered protected.
(b) Quantification of toxin-neutralizing activity: The activities of HmAb were quantitated by the method routinely used for TIG, using standardized doses of toxin at L+/20 or L+/200 levels. Purified HmAb were titrated and mixed with toxin solution (450 MLD or 45 MLD). A volume of 0.4 ml of the mixtures was injected per mouse. The neutralizing activity is expressed as International Units (IU) per 100 µg IgG.
(c) In vivo fragment absorption assay: Samples of 250 µl containing polyclonal or monoclonal antibodies and various amounts of purified TT fragments (A or C) were incubated for 30 min. at 37°C. Afterwards 250 µl containing 25 µg purified TT was added to each sample, followed by an incubation for 30 min. at 37°C. Mice were injected with 400 µl. In each experiment controls included the administration of TT only, and of the corresponding antibody and toxin in the absence of fragments.

As shown in Table II, the neutralizing activities of the HmAb were determined according to the WHO procedure routinely used for polyclonal TIG (20) using doses of toxin at L+/20 or L+/200 levels corresponding to 450 MLD and 45 MLD, respectively. The activities range from <0.5 to 13.2 IU/199µg IgG. The values obtained using 45 MLD and 450 MLD of TT are not consistent for ST17, ST11, and ST14. The injection of TT together with A-fragment specific HmAb caused a delay in the death of mice up to 6 days but never resulted in a complete long lasting protection. In contrast, mice injected with a mixture of the bi-specific HmAb ST12 or ST19, which recognize both the A- and C-fragments, and 450 MLD of toxin resulted in a complete protection (survival >28 days). Control mice receiving 450 MLD of TT died within 12 hrs. The protective capacity of a single HmAb, combinations of HmAb, or TIG against challenge with 450 MLD of TT is shown in Table III infra. ST15 exhibits the highest IU/weight values of the neutralizing, A-fragment specific HmAb (Table II), but independent of the antibody concentration does not confer full protection over 28 days (Table III infra). The combination of the two A-fragment specific HmAb ST15 and ST11 is able to provide full protection, whereas the application of the combination of ST15 and ST17 results only in a delay of death. In contrast, ST12 alone or combined with one or two A-fragment specific HmAb is comparable in the protectivity to TIG, Table III:

**Table III**

| PROTECTIVE CAPACITIES OF SINGLE HMAB, COMBINATIONS OF HMAB AND OF TIG | | | | | |
|---|---|---|---|---|---|
| preparations^{a} | mortality (days of survival) IU/ml | | | | |
| | 0.5 | 1.12 | 2.5 | 5.7 | 12.9 |
| ST15 | 5.5 | 5.5 | 5.5 | 3.5 | 0.5 |
| ST15/ST11 | - | - | - | 6 | 0.5 |
| ST15/ST17 | 14 | 9 | 9 | 6 | 0.5 |
| ST12 | - | 6.5 | 5.5 | 3.5 | 2.5 |
| ST12/ST15 | - | - | - | 5.5 | 1 |
| TIG | - | - | - | 1 | 0.5 |

| | | | | | |
|---|---|---|---|---|---|
| ^{a} Purified HmAb and mixtures of equal amounts of each HmAb were used. A stock solution of TIG (Tetuman) containing 430 IU/ml was used in a dilution of 1 to 100. | | | | | |

The synergistic effect resulting from the mixing of ST12 with additional HmAb is shown in Figure 1. On a weight basis (IU/100µg IgG) the highest values are reached with combinations of two HmAb. The neutralizing activity of the mixture of ST12 and ST15 was 43 IU/100µg IgG.
(H) Characterization of TIG
In order to assess whether the isolated HmAb are representative for the antibodies in polyclonal preparations conferring protection, three independent Human tetanus immune globulins (TIG) were analyzed. These TIGs were obtained from the Swiss Serum and Vaccine Institute, Berne, Switzerland, (Tetuman^{R}), Immuno, Vienna, Austria (Tetabulin^{R}), and BEhring, Marburg, Germany (Tetagam^{R}S).
All three preparations exhibited similar binding patterns (Table IV).

**Table IV**

| BINDING PROPERTIES OF POLYCLONAL HYPERIMMUNE PREPARATIONS | | | | | | |
|---|---|---|---|---|---|---|
| preparation | titer (x10⁶) | | | aK (x10⁹ M⁻¹) | | |
| | TT | Fr.A | Fr.C | TT | Fr.A | Fr.C |
| Tetuman | 2.70 | 2.66 | 0.240 | 3.7 | 0.82 | 3.3 |
| Tetabulin | 1.62 | 2.17 | 0.520 | 3.9 | 0.88 | 4.1 |
| Tetagam S | 1.61 | 2.18 | 0.492 | 4.4 | 1.5 | 5.6 |

Antibody titers for TT and the A-fragment were similar and titers for the C-fragment were lower. Average affinities were similar for TT and the C-fragment and slightly lower for the A-fragment (Table IV).
In an attempt to distinguish between polyclonal antibodies specific for one fragment (A or C) and two fragments (A and C), TIG were preabsorbed with an excess of either A- or C-fragment and afterwards tested for their ability to bind to A- or C-fragment (Table V).

**Table V**

| CHARACTERIZATION OF FRAGMENT RECOGNITION | | | |
|---|---|---|---|
| antibody | solid phase | inhibition of binding (%) of control by preabsorption with | |
| | | Fragment A | Fragment C |
| ST15 | Fragment A | >95 | 0 |
| | Fragment C | N.B. | N.B. |
| ST12 | Fragment A | >99 | >97 |
| | Fragment C | >99 | >97 |
| Tetuman | Fragment A | 97 | 46 |
| | Fragment C | >88 | >88 |
| (N.B.), no binding. | | | |

Using HmAb as controls, the binding of the A-fragment specific HmAb ST15 is completely abolished by the preabsorption with A-fragment, whereas preabsorption with the same amount of C-fragment has no effect. the binding of ST12 to both the A- and the C-fragment can be completely abolished by preabsorption with either A- or C-fragment. Preabsorption with the C-fragment completely abolishes the binding to C-fragment, whereas 54% of the binding to the A-fragment remains. These results suggest that TIG dominantly contains antibodies specific for the A-fragment, less antibodies specific for both fragments, and relatively few C-fragment specific antibodies. This is consistent with the titers on purified A- and C-fragment (Table IV).
In order to evaluate the effect of binding to moieties of the A-fragment and the C-fragment in terms of TT neutralization in vivo, we analyzed the capacity of fragments to inhibit antibody mediated protection against TT (Fig. 2). Preabsorption of the A-fragment specific HmAb ST15 with A-fragment results in a complete loss of protective capacity, however, not with C-fragment (Fig. 2A). Protection by ST12 can be diminished by preabsorption with both A-fragment or C-fragment, whereas the A-fragment is a more potent inhibitor (Fig. 2B). Protection of all three TIG was completely inhibited by the preabsorption with 4µg C-fragment did not affect protection by TIG. These results suggest that binding to the A-fragment is more important than binding to the C-fragment for the antibody mediated neutralization of TT in vivo.
(I) Discussion
The above experiments demonstrate that the human monoclonal anti-tetanus toxin antibodies of the present invention are capable of fully replacing polyclonal antibody preparations which are currently used for the prophylaxis and treatment of tetanus. The experiments also demonstrate that A-fragment specific antibodies play a major role in mediating protection.
The five HmAb exhibiting high antitoxic activity in vivo were specific for the A-fragment. Two HmAb exhibiting high antitoxic activity in vivo bound to both, the A-fragment and the C-fragment.
The best protection provided by one HmAb was 13.2 IU/100µg IgG. On a weight basis, the best protection was achieved with a combination of two HmAb (43 IU/100µg IgG).
Thus, the present invention for the first time permits to substitute a polyclonal dose of 250 IU, containing 100-170 mg IgG, by a monoclonal preparation only containing 0.7 mg of a mixture of two HmAb, see Table VI:

**Table VI**

| REQUIREMENTS FOR THE GENERATION OF AN IMMUNOPROTECTIVE DOSE BY POLYCLONAL AND BY MONOCLONAL ANTIBODIES | | |
|---|---|---|
| | Polyclonal preparation | Monoclonal preparation |
| International units (IU) | 250 | 250 |
| Purified antibodies (mg) | 100 - 170 | 0.7 |

## Claims

1. A monoclonal anti-tetanus toxin antibody binding to the light chain of the tetanus toxin and neutralizing the biological activity of the tetanus toxin wherein said antibody has a neutralizing capacity of at least 2 IU/100µg Ig, preferably of at least 4 IU/100µg Ig.

2. The monoclonal anti-tetanus toxin antibody according to claim i which has a neutralizing capacity of at least 10 IU/100µg Ig.

3. The monoclonal anti-tetanus toxin antibody according to claim 1 or 2 which is a human antibody.

4. A monoclonal anti-tetanus toxin antibody binding to the light chain of the tetanus toxin and neutralizing the biological activity of the tetanus toxin wherein said antibody additionally binds to the C-fragment of the heavy chain of the tetanus toxin.

5. The monoclonal anti-tetanus toxin antibody according to any one of claims 1 to 4 which has an affinity constant (aK) to said tetanus toxin of at least 1 x 10⁹ M⁻¹.

6. The monoclonal anti-tetanus toxin antibody according to claim 5 which has an affinity constant (aK) to said tetanus toxin of at least 5 x 10⁹ M⁻¹.

7. The monoclonal anti-tetanus toxin antibody according to any one of claims 1 to 6 which is an antibody of the class IgG, preferably IgG1.

8. The monoclonal anti-tetanus toxin antibody according to any one of claims 1 to 7 which is a chimeric, a humanized or a bi-specific rearranged monoclonal antibody.

9. A cell line producing a monoclonal anti-tetanus toxin antibody according to any one of claims 1 to 7.

10. The cell line according to claim 9 which is a hybridoma.

11. A method for the production of a monoclonal anti-tetanus toxin antibody according to any one of claims 1 to 8, comprising the cultivation of a cell line acccording to claim 9 or 10 and the isolation of the antibody from the culture.

12. A pharmaceutical composition containing at least one, preferably at least two monoclonal antibodies according to any one of claims 1 to 8 optionally in admixture with a pharmaceutically acceptable carrier and/or diluent.

13. The pharmaceutical composition according to claim 12 which contains a monoclonal anti-tetanus toxin antibody according to any one of claims 1 to 8 which binds to the light chain of the tetanus toxin and in addition a monoclonal anti-tetanus toxin antibody according to any one of claims 4 to 8 which binds to the light chain of the tetanus toxin and also to the C-fragment of the heavy chain of the tetanus toxin.

14. The pharmaceutical composition according to claim 12 or 13 for the passive immunization against tetanus.

15. The pharmaceutical composition according to any one of claims 12 to 14 which contains each of the monoclonal antibodies in an amount of at least 0,7 mg Ig corresponding to an immunoprotective dosage of at least 250 IU.

16. The pharmaceutical composition according to claim 13, wherein said combined monoclonal antibodies have a neutralizing capacity of 43 IU/100µg Ig.

17. A method for the preparation of a pharmaceutical composition according to any one of claims 12 to 16 which comprises combining at least one, preferably at least two monoclonal antibodies according to any one of claims 1 to 8, with a pharmaceutically acceptable carrier and/or diluent.

## Patentansprüche

1. Monoclonaler Anti-Tetanustoxin-Antikörper, der an die leichte Kette des Tetanustoxins bindet und die biologische Aktivität des Tetanustoxins neutralisiert, wobei der Antikörper eine Neutralisierungskapazität von mindestens 2 IU/100µg Ig, vorzugsweise mindestens 4 IU/100µg Ig besitzt.

2. Monoclonaler Anti-Tetanustoxin-Antikörper nach Anspruch 1, der eine Neutralisierungskapazität von mindestens 10 IU/100µg Ig besitzt.

3. Monoclonaler Anti-Tetanustoxin-Antikörper nach Anspruch 1 oder 2, der ein menschlicher Antikörper ist.

4. Monoclonaler Anti-Tetanustoxin-Antikörper, der an die leichte Kette des Tetanustoxins bindet und die biologische Aktivität des Tetanustoxins neutralisiert, wobei der Antikörper zusätzlich an das C-Fragment der schweren Kette desTetanustoxins bindet.

5. Monoclonaler Anti-Tetanustoxin-Antikörper nach einem der Ansprüche 1 bis 4, der eine Affinitätskonstante (aK) für das Tetanustoxin von mindestens 1x10⁹ M⁻¹ besitzt.

6. Monoclonaler Anti-Tetanustoxin-Antikörper nach Anspruch 5, der eine Affinitätskonstante (aK) für das Tetanustoxin von mindestens 5x10⁹ M⁻¹ besitzt.

7. Monoclonaler Anti-Tetanustoxin-Antikörper nach einem der Ansprüche 1 bis 6, der ein Antikörper der IgG-Klasse, vorzugsweise IgG1 ist.

8. Monoclonaler Anti-Tetanustoxin-Antikörper nach einem der Ansprüche 1 bis 7, der ein chimärer, ein humanisierter oder ein bi-spezifischer veränderter monoclonaler Antikörper ist.

9. Zellinie, die einen monoclonalen Anti-Tetanustoxin-Antikörper nach einem der Ansprüche 1 bis 7 produziert.

10. Zellinie nach Anspruch 9, die ein Hybridom ist.

11. Verfahren zur Herstellung eines monoclonalen Anti-Tetanustoxin-Antikörpers nach einem der Ansprüche 1 bis 8, das die Züchtung einer Zellinie nach Anspruch 9 oder 10 und die Isolierung des Antikörpers aus der Kultur umfaßt.

12. Arzneimittel, das mindestens einen, vorzugsweise mindestens zwei monoclonale Antikörper nach einem der Ansprüche 1 bis 8, gegebenenfalls im Gemisch mit einem pharmazeutisch verträglichen Träger und/oder Verdünnungsmittel enthält.

13. Arzneimittel nach Anspruch 12, das einen monoclonalen Anti-Tetanustoxin-Antikörper nach einem der Ansprüche 1 bis 8 enthält, der an die leichte Kette des Tetanustoxins bindet, und zusätzlich einen monoclonalen Anti-Tetanustoxin-Antikörper nach einem der Ansprüche 4 bis 8, der an die leichte Kette des Tetanustoxins und auch an das C-Fragment der schweren Kette des Tetanustoxins bindet.

14. Arzneimittel nach Anspruch 12 oder 13 für die passive Immunisierung gegen Tetanus.

15. Arzneimittel nach einem der Ansprüche 12 bis 14, das jeden der monoclonalen Antikörper in einer Menge von mindestens 0,7mg Ig entsprechend einer immunprotektiven Dosierung von mindestens 250 IU enthält.

16. Arzneimittel nach Anspruch 13, wobei die kombinierten monoclonalen Antikörper eine Neutralisierungskapazität von 43 IU/100µg Ig besitzen.

17. Verfahren zur Herstellung eines Arzneimittels nach einem der Ansprüche 12 bis 16, das das Kombinieren von mindestens einem, vorzugsweise mindestens zwei monoclonalen Antikörpern nach einem der Ansprüche 1 bis 8 mit einem pharmazeutisch verträglichen Träger und/oder Verdünnungsmittel umfaßt.

## Revendications

1. Anticorps anti-tétanotoxine monoclonal se liant à la chaîne légère de la tétanotoxine et neutralisant l'activité biologique de la tétanotoxine, dans lequel ledit anticorps a une capacité neutralisante d'au moins 2 UI/100 µg d'Ig, avantageusement d'au moins 4 UI/100 µg d'lg.

2. Anticorps anti-tétanotoxine monoclonal suivant la revendication 1, qui a une capacité neutralisante d'au moins 10 UI/100 µg d'Ig.

3. Anticorps anti-tétanotoxine monoclonal suivant l'une ou l'autre des revendications 1 et 2, qui est un anticorps humain.

4. Anticorps anti-tétanotoxine monoclonal se liant à la chaîne légère de la tétanotoxine et neutralisant l'activité biologique de la tétanotoxine, dans lequel ledit anticorps se lie de plus au fragment C de la chaîne lourde de la tétanotoxine.

5. Anticorps anti-tétanotoxine monoclonal suivant l'une quelconque des revendications 1 à 4, qui a une constante d'affinité (aK) à ladite tétanotoxine d'au moins 1 x 10⁹ M⁻¹.

6. Anticorps anti-tétanotoxine monoclonal suivant la revendication 5, qui a une constante d'affinité (aK) à ladite tétanotoxine d'au moins 5 x 10⁹ M⁻¹.

7. Anticorps anti-tétanotoxine monoclonal suivant l'une quelconque des revendications 1 à 6, qui est un anticorps de la classe IgG, avantageusement IgG1.

8. Anticorps anti-tétanotoxine monoclonal suivant l'une quelconque des revendications 1 à 7, qui est un anticorps chimérique, un anticorps humanisé ou un anticorps monoclonal réagencé bispécifique.

9. Lignée cellulaire produisant un anticorps anti-tétanotoxine monoclonal suivant l'une quelconque des revendications 1 à 7.

10. Lignée cellulaire suivant la revendication 9, qui est un hybridome.

11. Procédé pour la production d'un anticorps anti-tétanotoxine monoclonal suivant l'une quelconque des revendications 1 à 8, comprenant la culture d'une lignée cellulaire suivant l'une ou l'autre des revendications 9 et 10 et l'isolement de l'anticorps de la culture.

12. Composition pharmaceutique contenant au moins un, avantageusement au moins deux anticorps monoclonaux suivant l'une quelconque des revendications 1 à 8, éventuellement en mélange avec un support et/ou diluant pharmaceutiquement acceptable.

13. Composition pharmaceutique suivant la revendication 12, qui contient un anticorps anti-tétanotoxine monoclonal suivant l'une quelconque des revendications 1 à 8 qui se lie à la chaîne légère de la tétanotoxine et de plus un anticorps anti-tétanotoxine monoclonal suivant l'une quelconque des revendications 4 à 8 qui se lie à la chaîne légère de la tétanotoxine et également au fragment C de la chaîne lourde de la tétanotoxine.

14. Composition pharmaceutique suivant l'une ou l'autre des revendications 12 et 13 pour l'immunisation passive contre le tétanos.

15. Composition pharmaceutique suivant l'une quelconque des revendications 12 à 14, qui contient chacun des anticorps monoclonaux en une quantité d'au moins 0,7 mg d'lg correspondant à un dosage immunoprotecteur d'au moins 250 Ul.

16. Composition pharmaceutique suivant la revendication 13, dans laquelle lesdits anticorps monoclonaux combinés ont une capacité neutralisante de 43 UI/100 µg d'lg.

17. Procédé pour la préparation d'une composition pharmaceutique suivant l'une quelconque des revendications 12 à 16, qui comprend la combinaison d'au moins un, avantageusement d'au moins deux anticorps monoclonaux suivant l'une quelconque des revendications 1 à 8, avec un support et/ou diluant pharmaceutiquement acceptable.
